(19) Europäisches Patentamt
European Patent Office
Office européen des brevets

(11) Numéro de publication : **0 155 591**
**B1**

(12) **FASCICULE DE BREVET EUROPÉEN**

(45) Date de publication du fascicule du brevet : **25.05.88**

(21) Numéro de dépôt : **85102513.0**

(22) Date de dépôt : **06.03.85**

(51) Int. Cl.⁴ : **C 07 C 33/12**, A 61 K 7/46, A 23 L 1/226// C07C29/136, C07C45/72, C07C49/21, C07C45/68, C07C47/225, C07C29/40, C07C29/14, C07C45/62, C07C45/29

(54) **Dérivés hydroxylés de l'aldéhyde campholénique, leur utilisation à titre d'ingrédients parfumants et composition parfumante les contenant.**

(30) Priorité : **23.03.84 CH 1473/84**

(43) Date de publication de la demande : **25.09.85 Bulletin 85/39**

(45) Mention de la délivrance du brevet : **25.05.88 Bulletin 88/21**

(84) Etats contractants désignés : **CH DE FR GB LI NL**

(56) Documents cités :
**GB-A- 2 024 208**
**US-A- 3 903 174**
**US-A- 4 170 577**
**US-A- 4 173 585**

(73) Titulaire : **FIRMENICH SA**
**1, route des Jeunes**
**CH-1211 Genève 8 (CH)**

(72) Inventeur : **Schulte-Elte, Karl-Heinrich, Dr.**
**44, chemin de Carabot**
**CH-1213 Onex (CH)**
Inventeur : **Müller, Bernard**
**16, route de Malagnou**
**CH-1208 Genève (CH)**
Inventeur : **Pamingle, Hervé**
**20, avenue Henri-Dunant**
**CH-1205 Genève (CH)**

(74) Mandataire : **Salvadori, Giuseppe, Dr.**
**c/o Firmenich S.A. Case Postale 239**
**CH-1211 Genève 8 (CH)**

**EP 0 155 591 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'huile de santal représente certainement de nos jours encore l'un des éléments les plus prisés par le parfumeur, non seulement à cause de ses caractères odorants propres, mais également en raison des propriétés fixatives qu'il exerce dans bon nombre de compositions de nature variée.

L'art antérieur fait état de produits de synthèse dont les propriétés odorantes peuvent être assimilées à celles montrées par l'essence naturelle.

Dans ce contexte, le brevet US no. 4 052 341 décrit une composition parfumante constituée par un mélange de 3-méthyl-5-(2,2,3-triméthylcyclopent-3-ène-1-yl)-pentane-3-ol et de 6-(2,2,3-triméthylcyclopent-3-ène-1-yl)-hexane-3-ol, deux alcools respectivement de formule

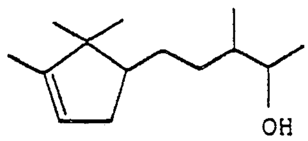

et

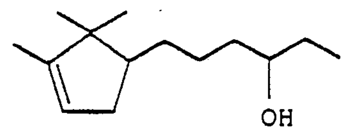

Le brevet RDA no. 68 936 décrit des composés de formule

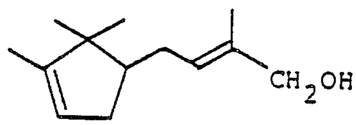

;

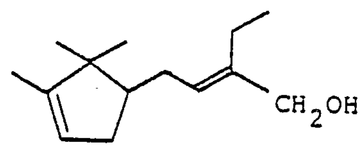

et

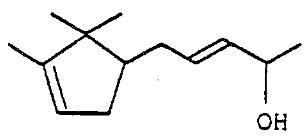

Les deux premiers composés possèdent un caractère odorant qui rappelle le musc et l'huile de santal, tandis que le troisième possède une odeur qui rappelle la figue avec toutefois une légère tonalité de bois de cèdre.

Une étude approfondie ayant trait aux composés odorants de type de bois de santal a été publiée par E.-J. Brunke et E. Klein [Essential Oils, Ed. B.D. Mookherjee et C.J. Mussinan, p. 83 et ss., Allured Publishing Corp. (1981)].

De cette étude, il apparaît qu'un des éléments de synthèse efficaces pour la préparation de tels composés est constitué par l'aldéhyde campholénique de formule

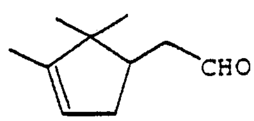

d'où la présence de cette unité moléculaire dans plusieurs composés de l'art antérieur.

Enfin, R.E. Naipawer et al. [Essential Oils, op. cit., p. 105 et ss] se sont attachés à mettre en lumière les relations pouvant exister entre la structure moléculaire et l'odeur de type bois de santal de certains parmi les composés connus. Une telle étude a mis en lumière les caractères « structuraux » auxquels, on pense, doivent obéir les composés chimiques pour qu'ils développent l'odeur recherchée.

Il est intéressant de remarquer à ce propos qu'aucun des composés examinés par Naipawer et al. ne possède une double substitution à l'atome de carbone en position 2 de la chaîne ouverte. D'autre part, aucun des composés décrits ne comporte une substitution à l'atome de carbone en position 4 de ladite chaîne.

Plusieurs spécialités, créées à base des composés précités de l'art antérieur, ont fait leur apparition sur le marché au cours des dernières années. Il s'agit en l'occurrence des composés suivants :

| Composé | Origine | Formule du constituant principal |
|---|---|---|
| SANDALORE | L. Givaudan | 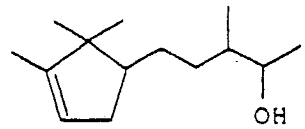 |
| SANDACORE | Kao Soap | 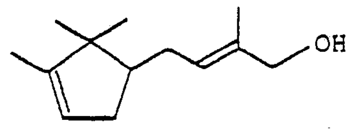 |
| BRAHMANOL | Dragoco | 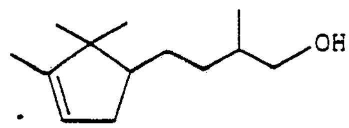 |
| BACDANOL | Int. Flavors & Fragrances | 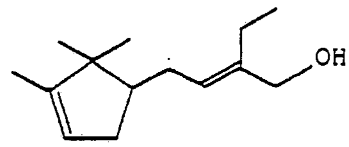 |

Au vu du nombre des composés déjà proposés, on aurait donc pu penser que le parfumeur possédait désormais, dans son activité de création, un choix suffisamment grand d'ingrédients santalés.

En réalité, il s'est avéré que tel n'était pas le cas. S'il est indéniable que chacun desdits composés de l'art antérieur est à même d'apporter, de par ses qualités, une note santalée aux compositions auxquelles il est incorporé, les effets obtenus varient fortement d'un composé à l'autre. Leur pouvoir de diffusion, par exemple, ainsi que leur substantivité peuvent également varier, et ce en fonction de l'application particulière à laquelle ils sont destinés. Quoiqu'ayant été tous définis comme des composés à odeur santalée, aucun d'entre eux ne présente une odeur identique. L'homme de l'art doit reconnaître en effet que la note typique de l'essence du bois de santal n'est que la résultante d'une variété de notes odorantes diverses qui s'apparentent tour à tour à celle du santalol, du bois de cèdre, de l'essence du bois de gaïac, ainsi qu'à des notes douces, balsamiques, légèrement ambrées, épicées, animales, voire de transpiration, ou de notes lactées de type lait bouilli. Dès lors, il n'est pas surprenant de constater qu'aucun des produits proposés par l'art antérieur ne peut à lui seul remplacer l'essence naturelle du bois de santal, chacun contribuant dans une mesure plus ou moins grande à l'un ou l'autre de ses caractère particuliers. Ce fait peut par ailleurs paraître étonnant lorsque l'on considère l'analogie de structure présentée entre eux par les dérivés connus de l'aldéhyde campholénique précités.

Ceci confirme une fois encore le caractère aléatoire de toute prévision dans le domaine des composés odorants pour lesquels, à ce jour, aucune théorie ne permet une corrélation exacte entre leur structure moléculaire et leur odeur.

Il faut encore mentionner que dans le cas des composés « santalés » cités, l'on a observé des effets d'anosmie marqués, plusieurs personnes, expertes ou non, étant dans l'impossibilité de percevoir l'odeur développée par l'un ou l'autre des composés qui leur étaient soumis. Tous ces phénomènes font qu'il existe toujours une nécessité impérieuse de développer des nouveaux composés à caractère santalé afin d'enrichir davantage la palette du parfumeur et de lui offrir des produits adéquats pour des applications variées.

La présente invention apporte une solution nouvelle à ce problème. Elle concerne en effet les composés de formule

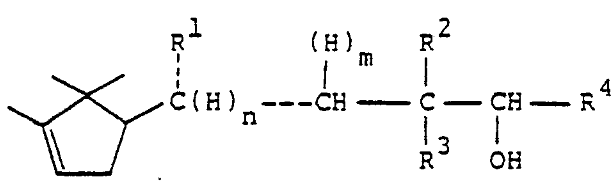

(I)

dans laquelle :

a. $R^1 = CH_3$ ; $n = m = 1$ ; $R^2 = CH_3$ ; $R_3 = R^4 = H$,
b. $R^1 = CH_2$ ; $n = 0$ ; $m = 1$ ; $R^2 = CH_3$ ; $R^3 = R^4 = H$,
c. $R^1 = CH\text{-}CH_3$ ; $n = 0$ ; $m = 1$ ; $R^2 = CH_3$ ; $R^3 = R^4 = H$,
d. $R^1 = H$ ; $n = m = 0$ ; $R^2 = R^4 = CH_3$ ; $R^3 = C_2H_5$,
e. $R^1 = H$ ; $n = m = 0$ ; $R^2 = R^3 = R^4 = CH_3$,
f. $R^1 = H$ ; $n = m = 0$ ; $R^2 = CH_3$ ; $R^3 = C_2H_5$ ; $R^4 = H$,
g. $R^1 = H$ ; $n = m = 0$ ; $R^2 = R^3 = CH_3$ ; $R^4 = H$,
h. $R^1 = H$ ; $n = m = 1$ ; $R^2 = CH_3$ ; $R^3 = C_2H_5$ ; $R^4 = H$,
i. $R^1 = H$ ; $n = m = 1$ ; $R^2 = R^3 = CH_3$ ; $R^4 = H$

et dans laquelle l'une des lignes pointillées indique une liaison simple carbone-carbone et l'autre une liaison double, ou chacune des deux indique une liaison simple.

La présente invention a trait également à l'utilisation desdits composés à titre d'ingrédients parfumants pour la préparation de parfums ou produits parfumés. Elle concerne en outre un parfum ou un produit parfumé résultant de ladite utilisation.

L'invention a enfin pour objet un procédé pour la préparation des composés de formule (I).

L'invention est définie comme indiqué aux revendications.

Nous avons découvert de façon surprenante que les composés (I) de l'invention possèdent des propriétés olfactives supérieures à celles montrées par les composés de l'art antérieur. Cette supériorité apparaît non seulement à l'égard de leur caractère odorant propre mais, et surtout, à l'égard de leur puissance olfactive et de leur substantivité. Ce fait peut, à première vue, paraître étonnant au vu de la similarité de structure existant entre les nouveaux composés (I) et ceux de l'état de la technique ; fait d'autant plus étonnant que nombreux ont été les groupes de recherche qui se sont penchés sur l'étude des composés santalés, notamment à structure campholénique.

Tout en étant caractérisés par une note commune qui rappelle l'essence du bois de santal, les différents composés de formule (I) possèdent chacun des caractéristiques olfactives propres à savoir balsamiques, lactées, boisées du type bois de cèdre. L'homme de l'art peut donc, en fonction de ces différentes nuances, choisir le composé qui sert au mieux à l'application spécifique envisagée. Le 2-méthyl-4-(2,2,3-triméthyl-3-cyclopentène-1-yl)-4-pentène-1-ol, par exemple, est le composé dont les caractères odorants s'apparentent au mieux à ceux de l'essence de santal naturelle. Le 3,3-diméthyl-5-(2,2,3-triméthyl-3-cyclopentène-1-yl)-4-pentène-2-ol, par contre, possède une note secondaire balsamique, douce et lactée, tandis que le 3-éthyl-3-méthyl-5-(2,2,3-triméthyl-3-cyclopentène-1-yl)-4-pentène-2-ol, par contre, possède une note secondaire balsamique, douce et lactée, tandis que le 3-éthyl-3-méthyl-5-(2,2,3-triméthyl-3-cyclopentène-1-yl)-4-pentène-2-ol montre une note boisée plus marquée qui rappelle le bois de cèdre ou l'essence du bois de santal non distillée.

Malgré ces différences, tous les composés de formule (I) s'avèrent être plus puissants et olfactivement plus stables, en d'autres termes plus performants que les composés analogues connus à ce jour. Il en résulte des avantages certains dans leur application, tant au point de vue des effets obtenus que de l'économie réalisée.

Comme il est souvent observé en parfumerie, les proportions dans lequelles les composés de l'invention peuvent être employés varient dans une gamme de valeurs assez étendue. Des concentrations de l'ordre de 0,1 % en poids peuvent être employées lors du parfumage d'articles divers tels les savons, les shampoings, les cosmétiques, les détergents, solides ou liquides, les désodorisants, corporels ou d'ambiance, ou les produits d'entretien.

Des concentrations supérieures conviennent à la préparation de parfums ou d'eaux de toilette. Bien entendu, de telles valeurs ne doivent pas être interprétées de manière restrictive car elles dépendent de la nature du produit que l'on désire parfumer, de celle des coingrédients présents dans une composition donnée et surtout de l'effet que l'on désire obtenir. Comme les composés de l'invention se marient parfaitement avec la plupart des ingrédients parfumants courants, l'énumération de ces derniers est ici superflue.

Les exemples particuliers qui suivent servent à montrer de façon non limitative quelques applications possibles. Les composés de l'invention sont préparés à l'aide de procédés originaux et économiques à partir d'aldéhyde campholénique, un dérivé de l'α-pinène, un sous-produit de l'essence de térébenthine. Les procédés employés sont illustrés à l'aide des schémas suivants.

(Voir Schémas p. 5.)

Schéma 1

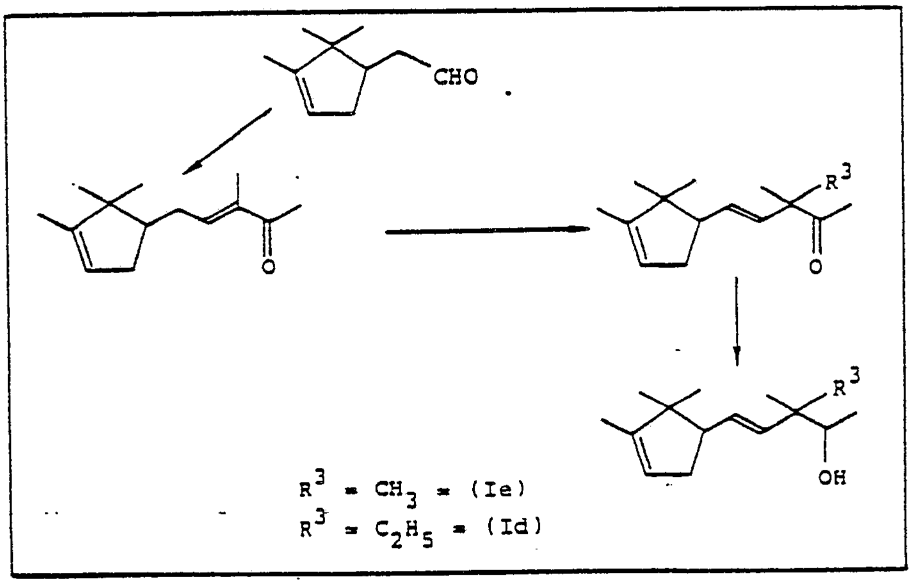


Schéma 2

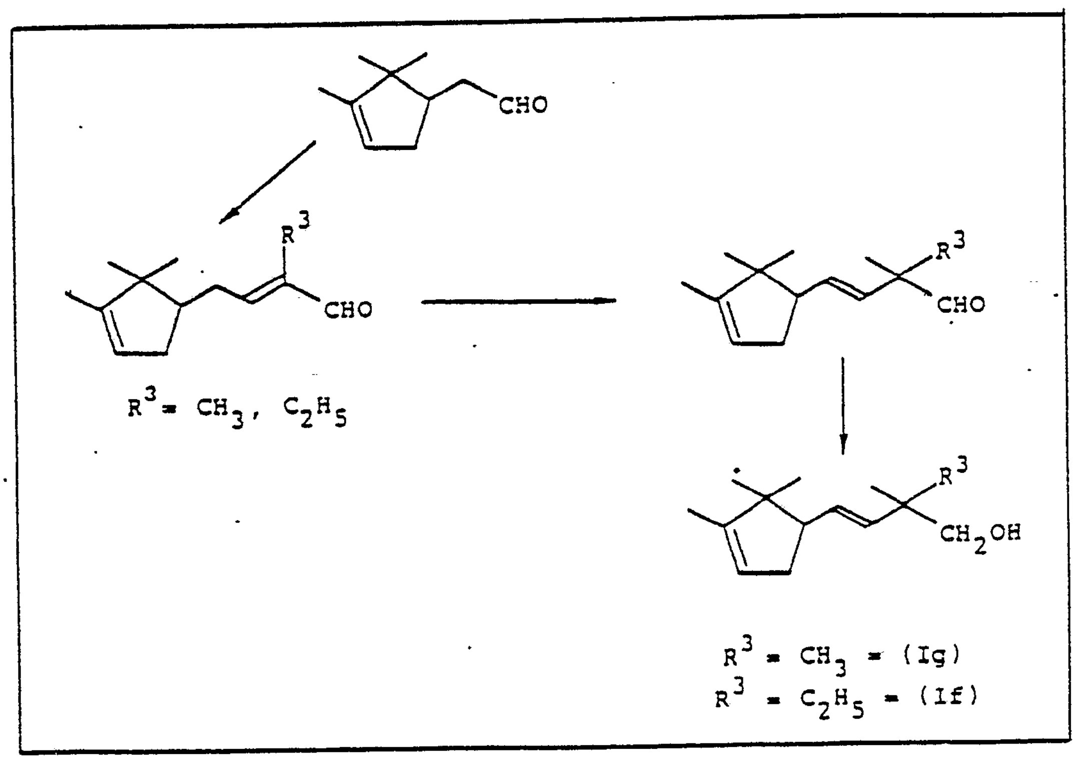

Schéma 3

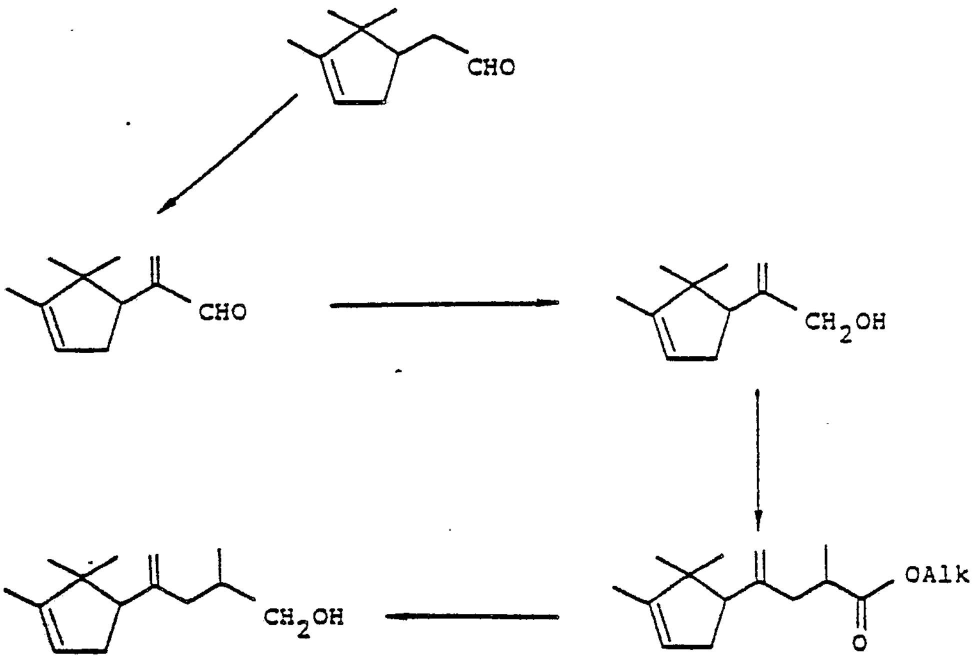

(Ib)

Schéma 4

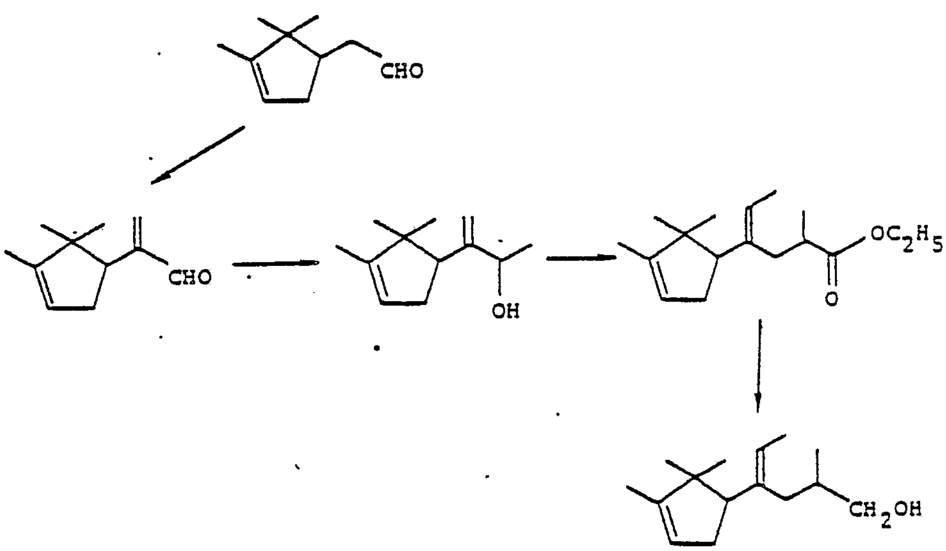

(Ic)

Schéma 5

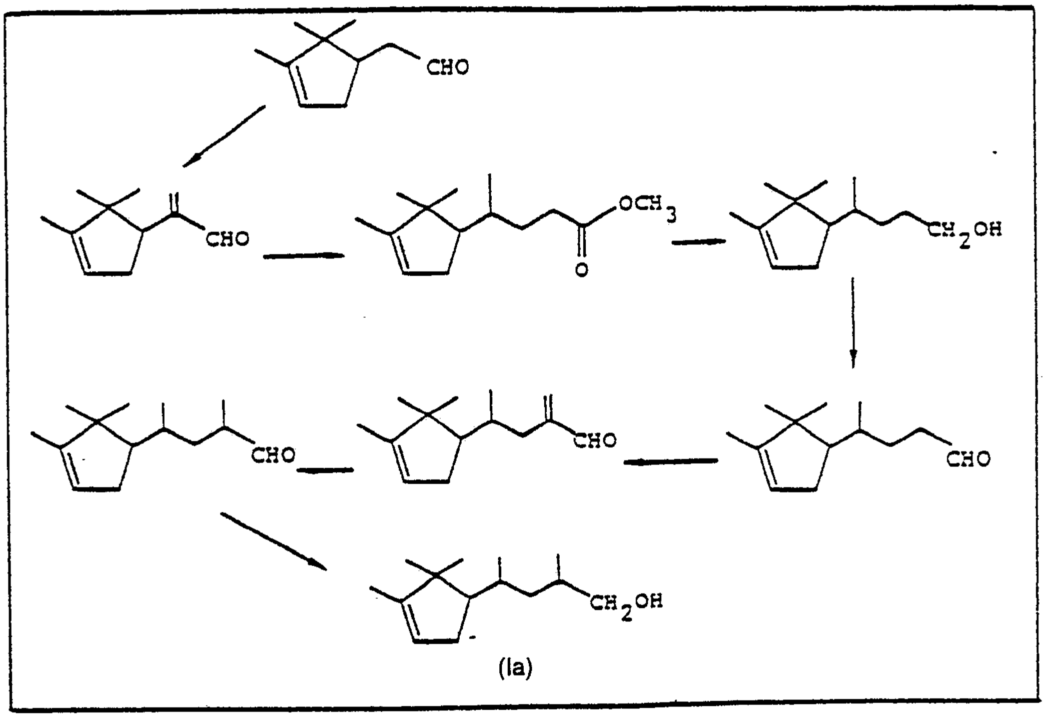

(Ia)

Il est connu que l'aldéhyde campholénique peut se présenter sous forme optiquement active ou sous forme d'un mélange quelconque de ses antipodes et ce en fonction de l'isomérie particulière de l'α-pinène utilisé comme produit de départ pour sa préparation. Ce fait implique que les produits issus des procédés illustrés plus haut peuvent se présenter sous différentes formes isomériques. Nous avons remarqué qu'au point de vue de leurs propriétés olfactives, les différents diastéréomères ne se différenciaient guère les uns des autres.

Par conséquent, lorsqu'il est fait mention dans la présente description d'un des composés de formule (I), l'on entend parler de l'un quelconque de ses diastéréomères ou de tous mélanges de ceux-ci.

Les méthodes particulières de synthèse utilisées pour la préparation des composés de l'invention, reposent sur l'application d'étapes discrètes conventionnelles. Le détail de ces procédés sera donné dans les exemples qui suivent. Il va sans dire que des modifications quant aux réactifs et aux conditions de réaction, par rapport à ceux qui sont décrits dans lesdits exemples, restent possibles, l'originalité des procédés étant basée sur le choix particulier des différentes étapes réactionnelles et leur enchaînement en vue d'obtenir les produits désirés.

L'invention est illustrée à l'aide des exemples suivants dans lesquelles les températures sont indiquées en degrés centigrades et les abréviations ont le sens usuel dans l'art.

## Exemple 1

Préparation de (-)-(E)-3,3-diméthyl-5-(2',2',3'-triméthyl-3'-cyclopentène-1'-yl)-4-pentène-2-ol

Une solution de 22,0 g (0,1 M) de 3,3-diméthyl-5-(2,2,3-triméthyl-3-cyclopentène-1-yl)-4-pentène-2-one dans 50 ml d'éthanol a été ajoutée goutte à goutte à un mélange de 2,85 g (75 mM) de borhydrure de sodium dans 100 ml d'éthanol. La réaction est légèrement exothermique et la température du mélange de réaction a été maintenue en dessous de 32° par refroidissement extérieur. Le mélange a été ensuite maintenu sous agitation pendant une nuit à température ambiante de l'excès d'éthanol a été évaporé sous pression réduite. Le résidu obtenu a été repris à l'éther, lavé à l'eau jusqu'à neutralité, séché et concentré pour fournir 23,7 g d'un produit bruit qui, par distillation fractionnée, a fourni 21,5 g de (-)-(E)-3,3-diméthyl-5-(2',2',3'-triméthyl-3'-cyclopentène-1'-yl)-4-pentène-2-ol(rend. 97%).

$[\alpha]^{20}_D = -11,8°$

IR : 3 400 $cm^{-1}$ ;

RMN (60 MHz ; $CDCl_3$) : 0,78 et 0,98 (6H, 2s) ; 1,02 (6H, s) ; 1,14 (3H, d, J = 3 Hz) ; 1,65 (3H, m) ; 3,35-3,68 (1H, m) ; 5,25 (1H, m) ; 5,38-5,55 (2H, m) δ ppm ;

SM : $M^+$ : 220(0,1) ; m/e : 204(1), 189(1), 178(21), 163(12), 149(7), 135(11), 121(34), 109(64), 91(15), 79(11), 69(100), 55(11), 41(27).

La 3,3-diméthyl-5-(2,2,3-triméthyl-3-cyclopentène-1-yl)-4-pentène-2-one, utilisée comme produit de départ dans le procédé décrit ci-dessus, peut être préparée ainsi.

a. (+)-(E)-3-Méthyl-5-(2',2',3'-triméthyl-3'-cyclopentène-1'-y1)-3-pentène-2-one

152 G (1,0 M) d'aldéhyde campholénique ayant une pureté de 75 % environ, ont été ajoutés goutte à goutte avec agitation en 15-20 min à un mélange, refroidi à — 5/— 10°, constitué par 400 g (6,1 M) de cétone méthyl-éthylique, 1 025 g (32 M) de méthanol, 30,5 g (0,54 M) d'hydroxyde de potassium et 380 g d'eau. Le mélange a été maintenu à — 5° pendant 24 h, puis à température ambiante pendant une même période, avant d'être neutralisé à l'aide d'$H_2SO_4$ à 62,5 %. Après concentration sous pression réduite, le résidu obtenu a été dissous dans 200 ml d'$H_2O$, repris à l'éther de pétrole 30/50, lavé jusqu'à neutralité, séché et concentré. On a ainsi obtenu 189,3 g d'un produit brut qui, par distillation à l'aide d'une colonne Vigreux de 15 cm, ont fourni 144,6 g de (E)-3-méthyl-5-(2,2,3-triméthyl-3-cyclopentène-1-yl)-3-pentène-2-one ayant une pureté de 86 % (rend. 60,4 %).

$[\alpha]^{20}_D$ = +2,2° ; Eb. 67°/6,65 Pa

IR : 1 670 $cm^{-1}$ ;

RMN (60 MHz ; $CDCl_3$) : 0,82 et 1,0 (6H, 2s) ; 1,65 (3H, s) ; 1,8 (3H, s) ; 2,28 (3H, s) ; 5,22 (1H, m) ; 6,56-6,83 (1H, m) δ ppm ;

SM : $M^+$ = 206(7) ; m/e : 191(16), 173(17), 158(6), 145(7), 136(39), 121(26), 109(49), 98(72), 93(67), 79(36), 67(61), 55(32), 43(100).

b. (-)-(E)-3,3-Diméthyl-5-(2',2',3'-triméthyl-3'-cyclopentène-1'-yl)-4-pentène-2-one

Dans un ballon contenant une solution de 45 g de NaOH dans 45 ml d'eau, sous atmosphère d'azote et à température ambiante, on a ajouté 0,8 g de bromure d'hexadécyl-triméthylammonium et, en une seule portion, 10,3 g (50 mM) de la cétone obtenue suivant le paragraphe a. ci-dessus d'une pureté de 89 % dans 10 ml de toluène. Le mélange de réaction a été vigoureusement agité, tandis qu'un courant de chlorure de méthyle y a été introduit. Après 9 heures environ, on a pu observer la disparition complète de la cétone de départ. Le tout a été versé sur de la glace, repris après 2 fractions d'éther et les extraits organiques ont été lavés jusqu'à neutralité, séchés, concentrés et distillés au moyen d'un four à boules (13,3 Pa). On a ainsi obtenu 9,3 g de la cétone désirée ayant une pureté de 92 % (rend. 87,4 %).

$[\alpha]^{20}_D$ = — 19,5° ; Eb. 50°/6,65 Pa

IR : 1 720 $cm^{-1}$ ;

RMN (60 MHz ; $CDCl_3$) : 0,78 et 0,99 (6H, 2s) ; 1,22 (6H, s) ; 1,65 (3H, s) ; 2,11 (3H, s), 5,12 (1H, m) ; 5,5-5,63 (2H, m) δ ppm ;

SM : $M^+$ = 220(4) ; m/e : 177(25), 162(2), 149(5), 135(5), 121(27), 109(53), 91(12), 69(100), 55(12), 41(22).

Exemple 2

Préparation de (-)-(E)-3,3-diméthyl-5-(2',2',3'-triméthyl-3'-cyclopentène-1'-yl)-4-pentène-2-ol

a. Un mélange de 456 g (3 M) d'aldéhyde campholénique ayant une pureté de 75 % et 348 g (6 M) d'aldéhyde propionique a été ajouté goutte à goutte à une solution à reflux de 18 ml d'hydroxyde de sodium à 30 % dans 800 ml d'éthanol. Une fois l'addition terminée, le mélange de réaction a été maintenu à reflux pendant 2 h, puis, après refroidissement à température ambiante, on a versé le tout sur 1 000 ml d'eau. Le produit a été extrait avec 3 fractions de 300 ml d'éther de pétrole 30/50 et les extraits organiques ont été lavés avec l'eau, puis avec une solution aqueuse de NaCl jusqu'à neutralité, séchés et concentrés. Le résidu a été distillé sur colonne Vigreux de 10 cm et a fourni 323 g de (+)-(E)-2-méthyl-4-(2',2',3'-triméthyl-3'-cyclopentène-1'-yl)-2-buténal (rend. 56 %).

$[\alpha]^{20}_D$ = + 1,2° ; Eb. 57°/9,31 Pa

IR : 2 735 et 1 740 $cm^{-1}$ ;

RMN (60 MHz ; $CDCl_3$) : 0,82 et 1,02 (6H, 2s) ; 1,65 (3H, m) ; 1,75 (3H, s) ; 5,22 (1H, m) ; 6,52 (1H, t, J = 6Hz) ; 9,4 (1H, s) δ ppm

SM : $M^+$ = 192(9) ; m/e : 177(5), 164(1), 159(16), 149(17), 121(40), 108(100), 93(88), 79(43), 67(49), 55(37), 41(48).

b. 19,2 g (0,1 M) de l'aldéhyde préparé suivant a. ci-dessus ont été ajoutés goutte à goutte à une solution de 13,4 g (0,12 M) de tert-butylate de potassium dans 100 ml de tert-butanol à 30°. La durée de l'introduction était d'environ 10 min, et le tout a été maintenu à cette température pendant une 1/2 h, puis il a été refroidi à 5°. A cette température, on y a ensuite introduit goutte à goutte 17 g (0,12 M) de iodure de méthyle et le mélange a été agité pendant 10 min à 5°, puis la température a été laissée remonter à température ambiante. Le mélange a été versé sur de l'eau glacée, il a été repris avec de l'éther de pétrole 30/50 et les extraits organiques ont été lavés jusqu'à neutralité, séchés et concentrés. Par distillation du résidu au moyen d'un four à boules (6,65 Pa), on a obtenu 15,4 g de (-)-(E)-diméthyl-4-(2',2',3'-triméthyl-3'-cyclopentène-1'-yl)3-buténal (rend. 75 %).

$[\alpha]^{20}_D$ = — 16,1°

IR : 1 740 et 2 740 $cm^{-1}$ ;

RMN (60 MHz ; $CDCl_3$) : 0,73 et 0,97 (6H, 2s) ; 1,18 (6H, s) ; 1,65 (3H, m) ; 5,25 (1H, m) ; 5,37-5,56 (2H, m) ; 9,32 (1H, s) δ ppm ;

SM : $M^+$ = 206(8) ; m/e 191(2), 177(24), 163(3), 149(5), 134(13), 121(28), 109(47), 91(18), 79(13), 69(100), 55(12), 41(32).

c. 3,09 G (15 mM) de l'aldéhyde obtenu suivant b. ci-dessus dans 15 ml d'éther anhydre ont été ajoutés à une solution de iodure de méthyle magnésium (préparée à partir de 2,6 g de iodure de méthyle et 0,43 g de magnésium en copeaux dans l'éther).

Le mélange de réaction a été maintenu sous agitation à température ambiante pendant 3 h, puis il a été versé sur une solution glacée saturée de $NH_4Cl$. Après extraction à l'éther, lavage des extraits éthérés avec $H_2O$/NaCl jusqu'à neutralité, séchage et concentration, on a obtenu par distillation au moyen d'un four à boules 3,15 g de (-)-(E)-3,3-diméthyl-5-(2',2',3'-triméthyl-3'-cyclopentène-1'-yl)-4-pentène-2-ol (rend. 95 %) dont les caractères analytiques étaient en tous points identiques à ceux d'un échantillon préparé conformément à l'Exemple précédent.

## Exemple 3

(-)-(1'S,E)-3-Ethyl-3-méthyl-5-(2',2',3'-triméthyl-3'-cyclopentène-1'-yl)-4-pentène-2-ol

Ce composé a été préparé conformément au procédé indiqué à l'Exemple 1 par réduction au moyen de borhydrure de sodium de (-)-(1'S,E)-3-éthyl-3-méthyl-5-(2',2',3'-triméthyl-3'-cyclopentène-1'-yl)-4-pentène2-one avec un rendement de 97 %.

$[\alpha]^{20}_D = -9,9°$

IR : 3 400 $cm^{-1}$ ;

RMN : 0,72 et 0,92 (9H, 2s) ; 0,9 (3H, t, J = 7Hz) ; 1,62 (3H, s) ; 5,22 (1H, m) ; 5,2-5,51 (2H, m) δ ppm ;

SM : $M^+$ = 236(0,1) ; m/e : 203(2), 192(32), 177(9), 163(6), 149(6), 135(14), 121(57), 109(100), 91(26), 83(92), 79(20), 67(21), 55(64), 41(27).

La (1'S,E)-3-éthyl-3-méthyl-5-(2',2',3'-triméthyl-3'-cyclopentène1'-yl)-4-pentène-2-one, utilisée comme produit de départ dans le procédé décrit ci-dessus, peut être obtenue par éthylation de (+)-E)-3-méthyl-5-(2',2',3'-triméthyl-3'-cyclopentène-1'-yl)-3-pentène-2-one (voir Exemple 1a.) avec du bromure d'éthyle selon la méthode suivante.

0,1 M de la penténone a été introduite goutte à goutte dans un ballon contenant une solution de 13,4 g (0,12 M) de tert-butylate de potassium dans 100 ml de tert-butanol à 30° (durée de l'introduction : 10 min). Le mélange a été laissé sous agitation pendant 30 min à 30°, puis il a été refroidi à 5° environ et à cette température on y a introduit goutte à goutte 0,12 M de l'halogénure d'alkyle choisi et le tout a été laissé sous agitation à cette température pendant 10 min, puis on le porte à température ambiante. Le mélange de réaction a été versé sur de l'eau glacée, extrait à l'éther de pétrole 30/50 et les extraits organiques ont été lavés, séchés et concentrés. Le produit désiré a été obtenu par distillation sous vide.

$[\alpha]^{20}_D = -8,9°$ ; rend. 32,5 %

IR : 1 700 $cm^{-1}$ ;

RMN (60 MHz ; $CDCl_3$) : 0,72 et 0,97 (6H, 2s) ; 1,17 (3H, s) ; 0,82 (3H, t, J = 7Hz) ; 1,63 (3H,s) ; 2,08 (3H, s) ; 5,23 (1H, m) ; 5,45-5,62 (2H, m) δ ppm ;

SM : $M^+$ = 234(4) ; m/e : 205(1), 191(42), 175(1), 163(3), 149(3), 135(10), 126(13), 121(42), 109(70), 91(15), 83(100), 79(12), 55(70), 43(29).

## Exemple 4

(+)-(1'S)-2-Méthyl-4-(2',2',3'-triméthyl-3'-cyclopentène-1'-yl)-1-pentanol

Pour la préparation de ce composé, on a suivi la méthode générale que voici.

Dans un ballon, muni d'un réfrigérant, d'un thermomètre et d'une ampoule à décanter, on a placé sous azote une suspension de 0,76 g (0,02 M) de $LiAlH^4$ dans 50 ml d'éther anhydre et on y a introduit goutte à goutte 0,04 M de (-)-(1'S)-2-méthyl-4-(2',2',3'-triméthyl-3'-cyclopentène-1'-yl)-pentanal en solution dans 80 ml d'éther anhydre, tout en maintenant au reflux. Le mélange de réaction a été ensuite agité à température ambiante pendant 2 h et on y a introduit avec précaution successivement : 1,52 ml de NaOH à 15 % et 4,56 ml d'eau. Après filtration, le filtrat clair a été évaporé sous vide et le résidu obtenu a été distillé au moyen d'un four à boules pour fournir le pentanol désiré (150°/13,3 Pa ; rend. 94 %).

$[\alpha]^{20}_D = +5,9°$

IR : 3 350 $cm^{-1}$ ;

RMN (60 MHz ; $CDCl_3$) : 0,78-1,15 (12H, m) ; 1,51-1,69 (3H, m) ; 3,22-3,62 (2H, m) ; 5,1-5,3 (1H, m) δ ppm ;

SM : $M^+$ = 210(14) ; m/e : 195(45), 177(10), 135(18), 121(55), 109(70), 99(65), 95(100), 91(22), 81(36), 67(42), 55(41), 41(35).

Le pentanal de départ peut être préparé ainsi.

a. 1 Mole de (-)-(1'S)-2-[2-(2',2',3'-triméthyl-3'-cyclopentène-1'-yl)-propyl]-2-propénal, voir b. ci-après, a été hydrogénée dans 1,5 l d'éthanol en présence de 4 g de nickel de Raney à température ambiante et à pression atmosphérique. Après absorption d'un équivalent d'$H_2$ on a arrêté la réaction, filtré, rincé, concentré et distillé. L'aldéhyde, obtenu avec un rendement de 96,5 %, présentait les caractères analytiques suivants :

$[\alpha]^{20}_D$ = — 2,5°

IR : 1 730 $cm^{-1}$ ;

RMN (60 MHz, $CDCl_3$) : 0,75-1,2 (12H, m) ; 1,5-1,7 (3H, m) ; 5,1-5,25 (1H, m) ; 9,47-9,66 (1H, m) δ ppm ;

SM : $M^+$ = 208(22) ; m/e : 193(28), 175(68), 166(11), 149(10), 135(82), 119(50), 109(100), 99(38), 91(42), 81(59), 67(68), 55(46), 43(73).

b. Le (-)-(1'S)-2-[2-(2',2',3'-triméthyl-3'-cyclopentène-1'-yl)-propyl]-2-propénal a été préparé par traitement de (-)-(1'S)-4-(2',2',3'-triméthyl-3'-cyclopentène-1'-yl)-pentanal voir c. ci-après avec du formaldéhyde. 9 G (46 mM) dudit pentanal ont été chauffés à reflux avec 15 g (46 mM) de formaldéhyde (solution à 30 %, origine Fluka A.G.) dans un appareil tricol muni de réfrigérant, thermomètre, agitateur, et ampoule à décanter. L'opération a lieu sous atmosphère d'azote. Tout en maintenant le reflux, on a ensuite ajouté rapidement 0,968 g (7,5 mM) de dibutylamine et on a laissé réagir pendant 3 h. Après refroidissement, la phase organique a été séparée par décantation et distillée. Le propénal désiré a été obtenu sous forme d'un mélange diastéréoisomérique (20/80) : 7,89 g d'une huile incolore ; Eb. 73°/20 Pa ; rend. 83 %.

$[\alpha]^{20}_D$ = — 11,7°

IR : 1 695 $cm^{-1}$ ;

RMN (60 MHz ; $CDCl_3$) : 0,75-0,1 (3H, m) ; 0,88 et 1,05 (6H, 2d) ; 1,48-1,58 (3H, m) ; 5,11-5,3 (1H, m) ; 6,02-6,2 (2H, 2s) ; 9,03 (1H, 1s) δ ppm ;

SM : $M^+$ = 206(13) ; m/e : 191(8), 173(17), 158(5), 145(5), 135(50), 131(12), 121(31), 109(100), 95(47), 91(26), 79(28), 67(30), 55(22), 41(39).

c. Le (-)-(1'S)-4-(2',2',3'-triméthyl-3'-cyclopentène-1'-yl)-pentanal a été préparé par oxydation de (-)-(1'S)-4-(2',2',3'-triméthyl-3'-cyclopentène-1'-yl)-1-pentanol, voir d. ci-après, à l'aide de chlorochromate de pyridinium. Une solution de l'alcool (0,66 M) dans 15 ml de $CH_2Cl_2$ anhydre a été ajoutée à une suspension de 22,8 g (0,106 M) de chlorochromate de pyridinium dans 150 ml de chlorure de méthylène. Après avoir été laissé à température ambiante pendant 1 h 1/2, le mélange réactionnel a été filtré sur une colonne remplie de $SiO_2$. Après concentration sous vide, le résidu formé a été distillé pour fournir l'aldéhyde désiré.

Eb. 65°/13,1 Pa ; rend. 75%

$[\alpha]^{20}_D$ = — 3,8°

IR : 1 720 $cm^{-1}$ ;

RMN (60 MHz ; $CDCl_3$) : 0,9 et 1,09 (6H, 2s) ; 0,85-1,2 (3H, m) ; 1,55-1,7 (3H, m) ; 5,12-5,3 (1H, m) ; 9,71-9,88 (1H, m) δ ppm ;

Le produit se présentait sous forme de deux diastéréomères :

(20 %) A : SM : $M^+$ = 194(35) ; m/e : 179(29), 161(100), 151(8), 145(2), 135(8), 119(80), 109(62), 95(59), 85(27), 81(41), 67(59), 55(32), 41(45).

(80 %) B : SM : $M^+$ = 194(23) ; m/e : 179(25), 161(100), 151(8), 135(58), 119(9), 109(71), 95(62), 91(34), 84(42), 81(49), 67(69), 55(40), 41(50).

d. Le (-)-(1'S)-4-(2',2',3'-triméthyl-3'-cyclopentène-1'-yl)-1-pentanol a été préparé à partir de (-)-(1'S)-4-(2',2',3'-triméthyl-3'-cyclopentène-1'-yl)-pentanoate de méthyle, voir e. ci-dessous, comme suit.

0,04 M de l'ester à réduire en solution dans 80 ml d'éther anhydre ont été introduites goutte à goutte à une suspension de $LiAlH_4$ (0,04 M ; 1,52 g) dans 100 ml d'éther anhydre tout en maintenant à reflux. Après avoir laissé le mélange sous agitation à température ambiante pendant 2 h, on y a ajouté successivement avec précaution : 1,52 ml d'eau, 1,52 ml de NaOH à 15 % et 4,56 ml d'eau. Après filtration, l'excès de solvants a été évaporé sous vide et le résidu a été distillé. Le pentanol obtenu se présentait sous forme d'un mélange de diastéréomères ayant Eb. 78°/6,65 Pa ; rend. 98 %.

$[\alpha]^{20}_D$ = — 4,2°

IR : 3 320 $cm^{-1}$ ;

RMN (60 MHz ; $CDCl_3$) : 0,85-1,2 (9H, m) ; 1,58-1,63 (3H, m) ; 3,61 (2H, t, J = 6Hz) ; 5,12-5,29 (1H, m) δ ppm ;

20 % diastéréomère A : SM : $M^+$ = 196(16) ; m/e : 181(100), 163(23), 152(2), 135(16), 121(45), 109(68), 95(97), 85(53), 81(48), 69(61), 61(1), 55(28), 41(59).

80 % diastéréomère B : SM : $M^+$ = 196(15) ; m/e : 181(65), 163(21), 153(4), 137(15), 121(38), 109(68), 95(100), 85(49), 81(41), 69(53), 55(25), 41(41).

e. Le (-)-(1'S)-4-(2',2',3'-triméthyl-3'-cyclopentène)-pentanoate de méthyle a été préparé à partir de (-)-(1'R)-2-(2',2',3'-triméthyl-3'-cyclopentène-1'-yl)-2-propénal, voir f. ci-dessous, comme suit.

1 M dudit propénal a été hydrogénée dans 1,5 l d'éthanol en présence de 4 g de nickel de Raney à température ambiante et à pression atmosphérique. Après absorption d'un équivalent d'hydrogène, la

réaction a été arrêtée et le mélange a été filtré, concentré et distillé pour fournir le (-)-(1'S)-2-(2',2',3'-triméthyl-3'-cyclopentène-1'-yl)-propanal, sous forme d'un mélange de diastéréomètres (60/40), ayant Eb. 47-52°/6,65 Pa, rend. 85%.

$[\alpha]^{20}_D$ = — 5,1°

IR : 1 730 $cm^{-1}$ ;

RMN (60 MHz ; $CDCl_3$) : 0,82-1,22 (9H, m) ; 1,5-1,68 (3H, m) ; 5,1-5,3 (1H, m) ; 9,51-9,65 (1H, m) δ ppm ;

A un mélange de 2 000 ml d'éther isopropylique anhydre, 150 g de HMPT et 24 g (1 M) d'hydrure de sodium maintenus sous atmosphère d'azote, on a progressivement ajouté 218 g (1,2 M) de diméthylphosphonoacétate de méthyle. La température du mélange réactionnel a été maintenue entre 20 et 25° durant l'addition, de même que lors de l'addition de 140 g (0,843 M) de l'aldéhyde obtenu ci-dessous dans 125 ml d'éther isopropylique. Après avoir chauffé pendant 3 heures à 70-72°, le mélange réactionnel a été finalement refroidi à température ambiante et l'excès d'hydrure de sodium décomposé par addition de méthanol. Après extraction à l'éther, lavage, séchage de la phase organique et évaporation des parties volatiles, on a recueilli 135 g de (+)-(1'S)-4-(2',2',3'-triméthyl-3'-cyclopentène-1'-yl)2-penténoate de méthyle avec un rendement d'environ 65 %. Le produit était constitué par un mélange de diastéréomères (20/80).

$[\alpha]^{20}_D$ = + 1°

IR : 1 720 $cm^{-1}$ ;

RMN (60 MHz ; $CDCl_3$) : 0,88 et 1,08 (6H, 2s) ; 0,81-1,2 (3H, m) ; 3,69 (3H, m) ; 5,09-5,28 (1H, m) ; 5,75 et 5,79 (1H, 2d, J = 16Hz) ; 6,87 et 6,91 (1H, 2dxd, J = 16 et 9Hz) δ ppm ;

Le penténoate obtenu a été réduit à l'aide d'une hydrogénation catalytique en présence de nickel de Raney suivant le procédé indiqué plus haut pour la réduction de (-)-(1'R)-2-(2',2',3'-triméthyl-3'-cyclopentène-1'-yl)-2-propénal. Le (-)-(1'S)-4-(2',2',3'-triméthyl-3'-cyclopentène-1'-yl)-pentanoate de méthyle présentait les caractères analytiques suivants : Eb. 56°/13,3 Pa ;

$[\alpha]^{20}_D$ = — 2,9°

IR : 1 740 $cm^{-1}$ ;

RMN (60 MHz ; $CDCl_3$) : 0,82 et 1,05 (6H, 2s) ; 0,78-1,03 (3H, m) ; 1,5-1,65 (3H, m) ; 3,67 (3H, s) ; 5,05-5,25 (1H, m) δ ppm ;

Le produit se présentait sous forme d'un mélange de diastéréomères (20/80).

A : SM : $M^+$ = 224(11) ; m/e : 209(100), 193(3), 177(50), 159(7), 149(18), 141(2), 135(63), 121(22), 115(15), 109(62), 95(33), 79(38), 73(15), 67(32), 55(32), 41(21).

B : SM : $M^+$ = 224(18) ; m/e : 209(100), 193(4), 177(61), 168(3), 159(8), 149(20), 135(81), 129(3), 121(29), 115(25), 109(98), 95(51), 91(31), 81(35), 73(22), 67(51), 59(21), 55(52), 41(36).

f. Le (-)-(1'R)-2-(2',2',3'-triméthyl-3'-cyclopentène-1'-yl)-2-propénal a été préparé à partir d'aldéhyde campholénique (pureté 75 % ; $[\alpha]^{20}_D$ = + 2,2°) suivant le procédé que voici : un mélange de 500 g (3,3 M) d'aldéhyde campholénique et 333 g (3,3 M) de formaldéhyde (solution à 30 % ; origine Fluka AG) a été chauffé à reflux sous atmosphère d'azote. 20 G (0,155 M) de dibutylamine ont été ensuite introduits rapidement dans le mélange, tout en maintenant la température à reflux. Le mélange réactionnel a été chauffé pendant 3 h, puis la phase organique a été décantée et distillée pour fournir 421,2 g du propénal désiré ayant Eb. 65-70°/4 × $10^2$ Pa (pureté 80 %) ; rend. 62,4 %.

$[\alpha]^{20}_D$ = — 75°

IR : 1 700 $cm^{-1}$ ;

RMN (60 MHz ; $CDCl_3$) : 0,69 et 1,02 (6H, 2s) ; 1,55-1,7 (3H, m) ; 2,15-2,5 (2H, m) ; 3,2 (1H, t, J = 8Hz) ; 5,18-5,4 (1H, m) ; 6,1 et 6,32 (2H, 2s) ; 9,55 (1H,s) δ ppm ;

SM : $M^+$ = 164(41) ; m/e : 149(75), 146(4), 135(27), 131(34), 121(66), 115(9), 107(95), 93(100), 79(71), 67(39), 53(42), 41(87).

Exemple 5

Préparation de (-)-(1'R)-2-méthyl-4-(2',2',3'-triméthyl-3'-cyclopentène-1'-yl)-4-hexène1-ol

Ce composé a été préparé par réduction à l'aide de $LiAlH_4$ de (-)-(1'R)-2-méthyl-4-(2',2',3'-triméthyl-3'-cyclopentène-1'-yl)-4-hexénoate d'éthyle suivant la méthode décrite à l'Exemple 4, paragraphe d.

L'alcool désiré a été obtenu sous forme d'un mélange d'isomères avec un rendement de 95 %.

$[\alpha]^{20}_D$ = — 60,2°

IR : 3 350 $cm^{-1}$ ;

RMN (60 MHz ; $CDCl_3$) : 0,7-1,12 (9H, m) ; 1,52-1,58 (6H, m) ; 3,31-3,62 (2H, m) ; 5,19-5,65 (2H, m) δ ppm ;

SM : $M^+$ = 222(43) ; m/e : 207(17), 193(10), 189(5), 175(5), 163(18), 149(47), 135(20), 121(100), 107(68), 93(76), 79(45), 67(39), 55(70), 41(93).

L'ester utilisé comme produit de départ dans ledit procédé peut être obtenu ainsi :

a. Le (-)-(1'R)-2-méthyl-4-(2',2',3'-triméthyl-3'-cyclopentène-1'-yl)-4-hexénoate d'éthyle a été préparé à partir de 0,3 M de (-)-(1'R)-3-(2',2',3-triméthyl-3'-cyclopentène-1'-yl)-3-butène-2-ol, voir b. ci-dessous, et

0,9 M d'orthopropionate d'éthyle. Le mélange des deux réactifs a été chauffé à 120°, puis 3 g d'acide pivalique dans 30 g d'ortho-porpionate ont été ajoutés au mélange réactionnel ; après 3 h, environ 50 ml d'éthanol ont été récupérés par évaporation directe du mélange. Après refroidissement, le mélange est distillé sous vide pour fournir l'ester désiré ayant Eb. 98°/6,65 Pa (rend. 96 %).

$[\alpha]^{20}_D$ = — 36,3°

IR : 1 740 $cm^{-1}$ ;

RMN (60 MHz ; $CDCl_3$) : 0,68-1,4 (12H, m) ; 1,52-1,8 (6H, m) ; 1,8-3,21 (6H, m) ; 3,5 (2H, q, J = 7Hz) ; 4,1 (2H, q, J = 7Hz) ; 5,20-5,33 (1H, m) ; 5,38 (1H, q, J = 7Hz) δ ppm ;

SM : $M^+$ = 264(64 ; m/e : 249(29), 235(52), 219(7), 208(28), 189(8), 175(42), 164(44), 149(66), 133(45), 119(61), 107(63), 91(64), 79(39), 67(31), 55(58), 41(100).

b. Le (-)-(1'R)-3-(2',2',3'-triméthyl-3'-cyclopentène-1'-yl)-3-butène-2-ol a été préparée à partir de (-)-(1'R)-2-(2',2',3'-triméthyl-3-'-cyclopentène-1'-y1)-2-propénal voir Exemple 4, f. par réaction du Grignard à l'aide de iodure de méthyle. L'alcool désiré a été obtenu avec un rendement de 85 %.

$[\alpha]^{20}_D$ = — 32°

IR : 3 350 $cm^{-1}$ ;

RMN (60 MHz ; $CDCl_3$) : 0,79 et 0,81 (3H, 2s) ; 1,07 (3H, s) ; 1,31 et 1,34 (3H, 2d, J = 6Hz) ; 1,58-1,72 (3H, m) ; 4,29 (1H, q, J = 7Hz) ; 4,93-5,02 (1H, m) ; 5,20-5,40 (2H, m) δ ppm ;

SM : $M^+$ =180(8) ; m/e : 162(17), 147(65), 137(6), 133(22), 119(100), 105(82),, 99(22), 91(78), 79(50) 67(41), 55(40), 43(69).

Exemple 6

Préparation de (-)-(1'S)-2-méthyl-4-(2',2',3'-triméthyl-3-'cyclopentène-1'-yl)-4-pentène-1-ol

Une solution de 53 g (0,212 M) de (-)-(1'S)-2-méthyl-4-(2',2',3'-triméthyl-3'-cyclopentène-1'-yl)-4-penténoate d'éthyle — voir a. ci-dessous — dans 200 ml d'éther anhydre a été introduite goutte à goutte sous bonne agitation et sous atmosphère d'azote à uns suspension de 7,6 g de $LiAlH_4$ (0,2 M) dans 300 ml d'éther anhydre tout en maintenant à reflux. Un fois l'addition terminée, le mélange réactionnel a été maintenu sous agitation à température ambiante, puis on y introduit successivement avec précaution : 7,6 ml d'eau, 7,6 ml de NaOH à 15 % et 22,8 ml d'eau. Après filtration, le solvant a été évaporé sous vide et le résidu distillé pour fournir 43,1 g d'une huile incolore ayant Eb. 76°/20 Pa (rend. 97,7 %).

$[\alpha]^{20}_D$ = — 52,7°

IR : 3 330 $cm^{-1}$ ;

RMN (60 MHz ; $CDCl_3$) : 0,75 et 1,09 (6H, 2s) ; 0,8-1,1 (3H, m) ; 1,52-1,69 (3H, m) ; 1,7-2,8 (6H, m) ; 3,31-3,62 (2H, m) ; 4,91 (2H, s) ; 5,18-5,35 (1H, m) δ ppm ;

Le produit se présentait sous forme d'un mélange de diastéréomères.

A : SM : $M^+$ = 208 (53) ; m/e : 193(36), 175(12), 161(3), 149(20), 135(85), 119(38), 107(100), 91(87), 79(40), 67(35), 55(31), 41(75).

B : SM : $M^+$ = 208(50) ; m/e : 193(32), 175(12), 149(18), 135(86), 119(41), 107(100), 99(15), 91(72), 79(46), 67(25), 55(32), 41(83).

a. Le (-)-(1'S)-2-méthyl-4-(2',2',3'-triméthyl-3'-cyclopentène-1'-yl)-4-pentéonate d'éthyle a été préparé à partir de (-)-(1'R)-2-(2',2',3'-triméthyl-3'-cyclopentène-1'-yl)-2-propène-1-ol — voir b. ci-après — ainsi. 55 G dudit alcool (0,33 M) et 158 g (0,9 M) d'ortho-propionate d'éthyle ont été chauffés à 120°, puis on y a introduit 3 g d'acide pivalique en solution dans 20 ml d'ortho-propionate d'éthyle. L'alcool éthylique formé (environ 50 ml) a été évaporé au fur et à mesure de sa formation (2 1/2 h).

Après refroidissement, on a distillé l'excès d'ortho-propionate d'éthyle sous vide et le résidu a été distillé.

Eb. 74-6°/4 Pa ; rend. 94 %.

$[\alpha]^{20}_D$ = — 46,2°

IR : 1 740 $cm^{-1}$ ;

RMN (60 MHz ; $CDCl_3$) : 0,75 et 1,08 (6H, 2s) ; 1,1-1,4 (6H, m) ; 1,56-1,70 (3H, m) ; 1,9-3,8 (6H, m) ; 4,11 (2H, q, J = 7Hz) ; 4,87 (2H, s) ; 5,18-5,32 (1H, m) δ ppm ;

SM : $M^+$ = 250(43) ; m/e : 235(40), 221(1), 205(4), 189(20), 176(8), 161(53), 149(38), 135(100), 121(33), 107(51), 91(43), 79(39), 67(17), 55(27), 41(52).

b. Le (-)-(1'R)-2-(2',2',3'-triméthyl-3'-cyclopentène-1'-yl)-2-propène-1-ol a été préparé à partir de (-)-(1'R)-2-(2',2',3'-triméthyl-3'-cyclopentène-1'-yl)-2-propène-1-al — voir Exemple 4, f.-. Une solution de 284 g (1,73 M) dudit aldéhyde (pureté 80 %) dans 0,9 l d'éther anhydre a été ajoutée goutte à goutte à une suspension maintenue en agitation et sous atmosphère d'azote de 26,2 g (0,69 M) de $LiAlH_4$ dans 1,8 l d'éther anhydre tout en maintenant la température du mélange de réaction à 0-5° (durée : 4 h).

Le mélange a été maintenu à 0° pendant 2 h sous agitation, puis on y a introduit successivement et avec précaution : 26,2 ml d'eau, 26,2 ml de NaOH à 15 % et 78,6 ml d'eau. Après 1 h, le mélange a été filtré,

concentré sous vide et le résidu obtenu a été distillé. On a ainsi obtenu 286 g d'une huile incolore de 80 % de pureté.

$[\alpha]^{20}_D$ = —56° : rend. 99 %.

IR : 3 330 $cm^{-1}$ ;

RMN (60 MHz) ; $CDCl_3$) ; 0,74 et 1,04 (6H, 2s) ; 1,5-1,62 (3H, m) ; 2,1-2,76 (3H, m) ; 3,95-4,2 (2H, m) ; 4,9-5,0 (1H, m) ; 5,15-5,35 (2H, m) δ ppm ;

SM : $M^+$ = 166(33) ; m/e : 151(7), 148(18), 133(100), 123(14), 119(18), 108(46), 105(82), 99(4), 91(74), 79(52), 67(35), 55(26), 41(42).

## Exemple 7

Préparation de (-)-(1'S,E)-2-éthyl-2-méthyl-4-(2',2',3'-triméthyl-3'-cyclopentène-1'-yl)-3-butène-1-ol.

Cet alcool a été préparé par réduction à l'aide de borydrure de sodium de l'aldéhyde correspondant. 0,1 M de (-)-(E)-2-éthyl-2-méthyl-4-(2',2',3'-triméthyl-3'-cyclopentène-1'-yl)-3-butène-1-al — voir a. ci-dessous — a été introduite goutte à goutte dans une solution de 2,85 g (75mM) de $NaBH_4$ dans 100 ml d'éthanol en veillant à ce que la température du melange ne dépasse pas 32°. Le mélange a été maintenu à température ambiante pendant une nuit sous agitation, puis l'éthanol a été évaporé sous vide.

Le résidu a été repris à l'éther, lavé jusqu'à neutralité, séché et concentré. Par distillation du résidu, on a obtenu avec un rendement de 95 % l'alcool désiré.

$[\alpha]^{20}_D$ = — 11,1°.

IR : 3 550 $cm^{-1}$.

RMN (60 MHz ; $CDCl_3$) : 0,72, 096 et 0,98 (9H, 3s) ; 0,82 (3H, t, J = 6Hz) ; 1,63 (3H, s) ; 3,31 (2H, d, J = 6Hz) ; 5,22 (1H, m) ; 5,28-5,49 (2H, m) δ ppm ;

SM : $M^+$ = 222(6) ; m/e : 207(1), 191(53), 161(3), 152(14), 135(18), 121(57), 109(95), 97(28), 83(100), 79(23), 67(21), 55(88), 41(35).

a. Le (-)-(E)-2-éthyl-2-méthyl-4-(2',2',3'-triméthyl-3'-cyclopentène-1'-yl)-3-butène-1al a été préparé par méthylation de (-)-(E)-2-éthyl-4-(2',2',3'-triméthyl-3'-cyclopentène-1'-yl)-2-butène-1-al, voir b. ci-dessous, suivant la méthode d'alkylation indiquée à l'exemple 1, b. Rend. 83 %.

$[\alpha]^{20}_D$ = — 8,5°.

Ir : 2 700, 1 710 $cm^{-1}$ ;

RMN (60 MHz ; $CDCl_3$) : 0,72 et 0,97 (6H, 2s) ; 1,14 (3H, s) ; 1,62 (3H, s) ; 5,21 (1H, m) ; 5,35-5,59 (2H, m) ; 9,33 (1H, s) δ ppm ;

SM : $M^+$ = 220(9) ; m/e : 205(1), 191(50), 163(4), 149(5), 135(13), 121(48), 109(72), 91(26), 83(100), 79(19), 67(17), 55(76), 41(30).

b. Le (-)-(E)-2-éthyl-4-(2',2',3'-triméthyl-3'-cyclopentène-1'-yl)-3-butène-1-al a été préparé à partir d'aldéhyde campholénique comme suit.

A un mélange maintenu à reflux de 3 ml de NaOH à 40 % dans 100 ml de méthanol, on a ajouté goutte à goutte un mélange de 76 g (0,5 M) de (+) aldéhyde campholénique (pureté 76 %) et 72 g (1 M) de butanal fraîchement distillé. La durée de l'introduction a été d'environ 30 min. Le mélange de réaction a été maintenu à reflux pendant 2 h, puis une fois revenu à température ambiante, il a été versé sur 350 ml d'eau et extrait avec 3 fractions chacune de 200 ml d'éther de pétrole 30/50.

Les extraits organiques ont été lavés à neutralité, séchés et concentrés pour fournir un résidu qui, par distillation sur colonne Vigreux, a donné 50,6 g (rend. 49 %) de l'aldéhyde désiré. Eb. 61°/6,65 Pa.

$[\alpha]^{20}_D$ = — 1,4°.

IR : 2 750, 1700 $cm^{-1}$ ;

RMN (60 MHz ; $CDCl_3$) : 0,83 et 1,01 (6H, 2s) ; 1,03 (3H, t, J = 7Hz) ; 1,65 (3H, s) ; 5,23 (1H, m) ; 6,47 (1H, t, J = 7Hz) ; 9,38 (1H, s) δ ppm ;

SM : $M^+$ 206(9) ; m/e : 191(4), 177(3), 173(8), 163(14), 136(22), 121(40), 108(100), 93(80), 79(39), 67(43), 55(25), 41(52).

## Exemple 8

Préparation de (-)-(1'S,E)-2,2-diméthyl-4-(2',2',3'-triméthyl-3'-cyclopentène-1'-yl)-3-butène-1-ol.

Ce composé a été obtenu conformément à la méthode décrite à l'exemple 7 à partir de (E)-2,2-diméthyl-4-(2',2',3'-triméthyl-3'-cyclopentène-1'-yl)-3-butène-1-al, lequel a son tour a été préparé à partir de (E)-2-méthyl-4-(2',2',3'-triméthyl-3'-cyclopentène-1'-yl)-2—butène-1-al.

Le composé désiré montrait les caractères analytiques suivants :

$[\alpha]^{20}_D$ = — 13,2°.

IR : 3 350 et 970 $cm^{-1}$ ;

RMN (60 MHz ; $CDCl_3$) : 0,72 et 0,93 (6H, 2s) ; 1,0 (6H, s) ; 1,62 (3H, large s) ; 3,3 (2H, d) ; 5,22 (1H, m) ; 5,31-5,52 (2H, m) δ ppm ;

SM : $M^+$ = 208(4) ; m/e : 193(3), 177(28), 161(2), 149(6), 138(19), 121(42), 109(57), 98(6), 93(24), 79(15), 69(100), 55(15), 41(28).

Exemple 9

Préparation de (-)-(1'S)-2-éthyl-2-méthyl-4-(2',2',3'-triméthyl-3'-cyclopentène-1'-yl)-1-butanol.

Ce composé a été préparé par réduction du butanal correspondant au moyen de $NaBH_4$ (voir méthode de l'exemple 7).
$[\alpha]^{20}_D$ = — 5,7°.
IR : 3 350 $cm^1$ ;
RMN : 0,78 et 0,98 (6H, 2s) ; 0,86 (3H, s) ; 1,64 (3H, large s) ; 3,36 (2H, s) ; 5,25 (1H, m) δ ppm ;
SM : $M^+$ = 224(9) ; m/e : 209(47), 191(26), 179(2), 149(7), 135(26), 121(76), 107(100), 95(75), 81(57), 67(56), 55(56), 41(32).

a. Le (-)-2-éthyl-2-méthyl-4-(2',2',3'-triméthyl-3'-cyclopentène-1'-yl)-butanal, utilisé comme produit de départ dans le procédé ci-dessus, a pu être obtenu par éthylation de (-)-2-méthyl-4-(2',2',3'-triméthyl-3'-cyclopentène-1'-yl)-butanal à l'aide de bromure d'éthyle.
$[\alpha]^{20}_D$ = — 7,1°.
IR : 2 700 et 1 730 $cm^{-1}$ ;
RMN (60 MHz ; $CDCl_3$) : 0,72 et 0,98 (6H, 2s) ; 1,02 (3H, s) ; 0,82 (3H, t) ; 1,63 (3H, large s) ; 5,23 (1H, m) ; 9,47 (1H, s) δ ppm ;
SM : $M^+$ = 222(18), m/e = 207(4), 189(10), 179(16), 147(4), 135(30), 121(78), 109(100), 95(63), 81(26), 67(30), 55(32), 41(38).

b. Le (-)-2-méthyl-4-(2',2',3'-triméthyl-3'-cyclopentène-1'-yl)-butanal a été obtenu par hydrogénation catalytique du (E)-2-méthyl-4-(2',2',3'-triméthyl-3'-cyclopentène-1'-yl)-2-buténal — voir exemple 2, § a. — en présence de nickel de Raney.
IR : 2 700 et 1 730 $cm^{-1}$ ;
RMN (60 MHz, $CDCl_3$) : 0,78 et 0,98 (6H, 2s) ; 1,13 (3H, d) ; 1,65 (H, large s) ; 5,21 (1H, m) ; 9,6 (1H, m) δ ppm ;
SM : $M^+$ = 194(25) : m/e : 179(20), 161(48), 151(8), 121(100), 109(54), 93(53), 79(28), 67(41), 55(21), 41(55).

Exemple 10

Préparation de (+)-2,2-diméthyl-4-(2',2',3'-triméthyl-3'-cyclopentène-1'-yl)-1-butanol.

Ce composé a été obtenu en suivant la méthode générale indiquée à l'exemple 9, par réduction au $NaBH_4$ de (+)-2,2-diméthyl-4-(2',2',3'-triméthyl-3'-cyclopentène-1'-yl)-butanal.
$[\alpha]^{20}_D$ = + 7,8°.
IR : 3 350 $cm^{-1}$ ;
RMN : 0,72 et 0,88 (6H, 2s) ; 0,98 (6H, 2s) ; 1,64 (3H, large s) ; 3,30 (2H, s) ; 5,22 (1H, m) δ ppm ;
SM : $M^+$ = 210(21) ; m/e : 195(100), 177(27), 165(3), 149(4), 135(14), 121(60), 107(83), 99(18), 95(54), 79(38), 67(38), 55(49), 41(80).

Exemple 11

Parfumage du savon

100 G de savon en copeaux, obtenu à partir d'une base de savon au sodium non parfumée préparée avec de l'huile de coco et de suif, ont été mélangés avec 0,5 g de l'un des composés préparés aux exemples 1 à 10 jusqu'à l'obtention d'une pâte homogène. La composition de savon ainsi obtenue possédait une odeur boisée, balsamique et santalée.

Exemple 12

Parfumage d'un détergent solide

Une base détergente solide a été préparée en mélangeant les ingrédients suivants (parties en poids) :

| | |
|---|---|
| Alkyl-benzène sulphonate lin. de sodium (longueur de la chaîne $C_{11-5}$) | 8,0 |
| Alcool de suif éthoxylé (14EO) | 2,9 |
| Savon au sodium (longueur de la chaîne $C_{12-16}$ 13-26 % ; $C_{18-22}$ 74-87 % | 3,5 |
| Triphosphate de sodium | 43,8 |

| | |
|---|---|
| Silicate de sodium | 7,5 |
| Silicate de magnésium | 1,9 |
| Carboxyméthylcellulose | 1,2 |
| Sodium EDTA | 0,2 |
| Sulphate de sodium | 21,2 |
| Eau | 9,8 |
| | 100,0 |

L'addition à un échantillon de la base détergente d'un des produits des exemples 1 à 10, à raison de 0,1 %, lui confère un caractère odorant distinct et élégant de type boisé, santalé.

Exemple 13

Une base adoucissante pour textiles a été préparée en mélangeant les ingrédients suivants (parties en poids) :

| Ingrédient | Parties | Origine |
|---|---|---|
| Praepagen WK | 10,0 | Hoechst |
| Emulsifiant O120 | 0,5 | Zschimmer & Schwarz |
| Polyglycol 400 | 2,0 | Hoechst |
| Eau distillée | 84,4 | |
| Colorant : | | |
| Brilliant Blau R 28032 sol. aqueuse à 0,5% | 0,1 | Siegle |
| Chlorure de sodium sol. aqueuse à 10% | 0,7 | |
| Poromycen F 10 | 0,1 | Kraft |
| Alcool isopropylique C+ | 2,0 | Shell |
| | 99,8 | |

L'addition à un échantillon de la base adoucissante indiquée ci-dessus d'un des produits des exemples 1 à 10, à raison de 0,2 %, lui confère une odeur boisée, santalée de grande finesse.

Exemple 14

Une composition parfumante de base de type boisé-animal a été préparé en mélangeant les ingrédients suivants (parties en poids) :

| | |
|---|---|
| Aldéhyde décylique à 10 %* | 10 |
| Aldéhyde undécylénique à 10 %* | 40 |
| Aldéhyde dodécylique à 10 %* | 10 |
| Aldéhyde de méthylnonylacétique à 10 %* | 10 |
| Essence de racines d'Angélique à 10 %* | 10 |
| Castoréum à 10 %* | 20 |
| Civette naturelle dégraissée à 10 %* | 10 |
| Galbanum résinoïde | 10 |
| Jasmin absolu 50 %* | 100 |
| Essence d'oliban | 10 |
| Patchouli | 30 |
| Acétate de styralyle | 15 |
| α-Isométhylionone | 95 |
| Essence de coriandre | 5 |

* dans le phtalate de diéthyle

| | |
|---|---|
| Hydroxycitronellol | 65 |
| Cyclopentadécanolide à 10 %* | 50 |
| Jasmin synthétique | 100 |
| Bergamote synthétique | 100 |
| Citron synthétique | 40 |
| Mousse de chêne absolu à 50 %* | 30 |
| Néroli synthétique | 20 |
| Muscone à 10 %* | 50 |
| Coumarine | 50 |
| Musc ambrette | 10 |
| Musc cétone | 30 |
| Phtalate de diéthyle | 50 |
| Total | 970 |

* dans le phtalate de diéthyle

Lorsqu'on ajoute à 98 g de la base décrite ci-dessus 2 g de (-)-(1'S,E)-3-éthyl-3-méthyl-5-(2',2',3'-triméthyl-3'-cyclopentène-1'-yl)-4-pentène-2-ol, l'on obtient une nouvelle composition dont le caractère de bois de santal est bien perceptible. En remplaçant ledit penténol par des quantités équivalentes de (-)-(1'S)-2-méthyl-4-(2',2',3'-triméthyl-3'-cyclopentène-1'-yl)-4-pentène-1-ol ou de (E)-3,3-diméthyl-5-(2,2,3-triméthyl-3-cyclopentène-1-yl)-4-pentène-2-ol, l'on obtient des compositions dont le caractère odorant est tout à fait similaire. Des effets similaires sont obtenus en remplaçant lesdits produits de synthèse par de l'essence de bois de santal, dans ce cas cependant la concentration a dû être augmentée à 3 %. En remplaçant par contre ledits produits de synthèse par une quantité équivalente de (+)-(1'S)-2-méthyl-4-(2',2',3'-triméthyl-3'-cyclopentène-1'-yl)-1-pentanol, on obtient une nouvelle composition possédant un caractère boisé, ambré.

## Exemple 15

Une composition parfumante de base de type fleuri-boisé a été préparée en mélangeant les ingrédients suivants (parties en poids) :

| | |
|---|---|
| Acétate de p-tert-butylcyclohexyle | 150 |
| Cédrénol | 100 |
| Essence d'orange Floride | 100 |
| Acétate de dihydroterpinyle | 70 |
| α-méthylionone[1)] | 60 |
| Essence de géranium synth. | 50 |
| Essence de lavande | 40 |
| Acétate de cédryle | 40 |
| Mayol®[1)2)] | 40 |
| Hydroxycitronellal[1)] | 40 |
| Musc cétone | 30 |
| Isoeugénol | 30 |
| Fixateur 404 10 %[1)3)]* | 30 |
| Acétate de myrcényle | 30 |
| Benzophénone | 20 |
| Salicylate d'amyle | 20 |
| Acétate de TCD[1)4)] | 20 |
| Acétate de cyclohexyléthanol | 20 |
| Mousse de chêne décolorée 50 %* | 20 |
| Méroli synth. | 10 |
| Dihydromyrcénol | 10 |
| β-Damascénone 1 %* | 10 |
| Essence de bergamote sans furocoumarine | 10 |
| Total | 950 |

* dans l'alcool éthylique à 95 %

1) origine : Firmenich SA, Genève

2) Hydroxyméthyl isopropyl cyclohexane

3) Ambrox® base

4) acétate de (tricyclo [5.2.1.$0^{2,6}$]déc-4-yl)-méthyle

La composition de base ainsi obtenue a été employée pour la préparation de nouvelles compositions. Pour ce faire, 5 g des produits sous-indiqués ont été ajoutés à 95 g de la composition de base et les compositions résultantes ont été soumises à une évaluation olfactive de la part d'un panel d'experts qui devaient se prononcer sur la qualité intrinsèque de l'odeur, son analogie avec l'essence de santal naturelle et sa puissance :

a. (-)-(1'S)-2-méthyl-4-(2',2',3'-triméthyl-3'-cyclopentène-1'-yl)-4-pentène-1-ol ;
b. (E)-3,3-diméthyl-5-(2',2',3'-triméthyl-3'-cyclopentène-1'-yl)-4-pentène-2-ol ;
c. Bacdanol (IFF)
d. Sandalore® (L. Givaudan)
e. Sandacore (Kao Soap)
f. Brahmanol® (Dragoco)

Compte tenu de ces critères d'évaluation, 80 % des experts se sont prononcés en faveur du (-)-(1'S)-2-méthyl-4-(2',2',3'-triméthyl-3'-cyclopentène-1'-yl)-4-pentène-1-ol comme étant le composé préféré, les 20 % restants ayant un avis partage entre les différents composés b. à f.

D'autre part, 60 % de ces mêmes experts ont placé le (E)-3,3-diméthyl-5-(2',2',3'-triméthyl-3'-cyclopentène-1'-yl)-4-pentène-2-ol en deuxième position.

## Revendications

1. Composés de formule

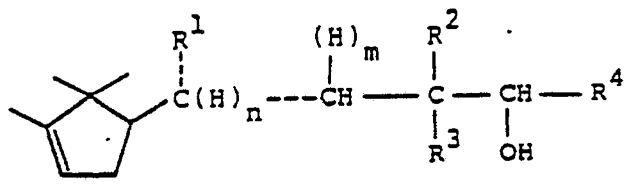

(I)

dans laquelle :

a. $R^1 = CH_3$ ; n = m = 1 ; $R^2 = CH_3$ ; $R^3 = R^4 = H$,
b. $R^1 = CH_2$ ; n = 0 ; m = 1 ; $R^2 = CH_3$ ; $R^3 = R^4 = H$,
c. $R^1 = CH\text{-}CH_3$ ; n = 0 ; m = 1 ; $R^2 = CH_3$ ; $R^3 = R^4 = H$,
d. $R^1 = H$ ; n = m = 0 ; $R^2 = R^4 = CH_3$ ; $R^3 = C_2H_5$,
e. $R^1 = H$ ; n = m = 0 ; $R^2 = R^3 = R^4 = CH_3$,
f. $R^1 = H$ ; n = m = 0 ; $R^2 = CH_3$ ; $R^3 = C_2H_5$ ; $R^4 = H$,
g. $R^1 = H$ ; n = m = 0 ; $R^2 = R^3 = CH_3$ ; $R^4 = H$,
h. $R^1 = H$ ; n = m = 1 ; $R^2 = CH_3$ ; $R^3 = C_2H_5$ ; $R^4 = H$,
i. $R^1 = H$ ; n = m = 1 ; $R^2 = R^3 = CH_3$ ; $R^4 = H$.

et dans laquelle l'une des lignes pointillées indique une liaison simple carbone-carbone et l'autre une liaison double, ou chacune des deux indique une liaison simple.

2. (-)-(E)-3,3-Diméthyl-5-(2',2',3'-triméthyl-3'-cyclopentène-1'-yl)-4-pentène-2-ol.
3. (-)-(1'S,E)-3-Ethyl-3-méthyl-5-(2',2',3'-triméthyl-3'-cyclopentène-1'-yl)-4-pentène-2-ol.
4. (+)-(1'S)-2-Méthyl-4-(2',2',3'-triméthyl-3'-cyclopentène-1'-yl)-1-pentanol.
5. (-)-(1'R)-2-Méthyl-4-(2',2',3'-triméthyl-3'-cyclopentène-1'-yl)-4-hexène-1-ol.
6. (-)-(1'S)-2-Méthyl-4-(2',2',3'-triméthyl-3'-cyclopentène-1'-yl)-4-pentène-1-ol.
7. (-)-(1'S,E)-2-Ethyl-2-méthyl-4-(2',2',3'-triméthyl-3'-cyclopentène-1'-yl)-butène-1-ol.
8. (-)-(1'S,E)-2,2-Diméthyl-4-(2',2',3'-triméthyl-3'-cyclopentène-1'-yl)-3-butène-1-ol.
9. (-)-(1',S)-2-Ethyl-2-méthyl-4-(2',2',3'-triméthyl-3'-cyclopentène-1'-yl)-1-butanol.
10. (+)-2,2-Diméthyl-4-(2',2',3'-triméthyl-3'-cyclopentène-1'-yl)-1-butanol.
11. Utilisation d'un des composés selon la revendication 1 à titre d'ingrédient parfumant pour la préparation de parfums et produits parfumés.
12. Composition parfumante résultant de l'utilisation selon la revendication 11.

## Claims

1. Compounds of formula

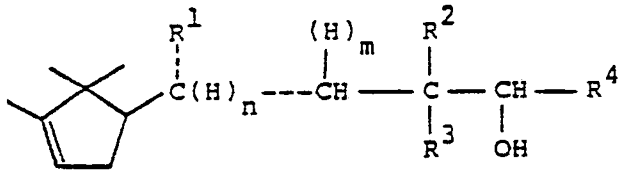

(I)

wherein :

a. $R^1 = CH_3$ ; n = m = 1 ; $R^2 = CH_3$ ; $R^3 = R^4 = H$,
b. $R^1 = CH_2$ ; n = 0 ; m = 1 ; $R^2 = CH_3$ ; $R^3 = R^4 = H$,
c. $R^1 = CH\text{-}CH_3$ ; n = 0 ; m = 1 ; $R^2 = CH_3$ ; $R^3 = R^4 = H$,
d. $R^1 = H$ ; n = m = 0 ; $R^2 = R^4 = CH_3$ ; $R^3 = C_2H_5$,
e. $R^1 = H$ ; n = m = 0 ; $R^2 = R^3 = R^4 = CH_3$,
f. $R^1 = H$ ; n = m = 0 ; $R^2 = CH_3$ ; $R^3 = C_2H_5$ ; $R^4 = H$,
g. $R^1 = H$ ; n = m = 0 ; $R^2 = R^3 = CH_3$ ; $R^4 = H$,
h. $R^1 = H$ ; n = m = 1 ; $R^2 = CH_3$ ; $R^3 = C_2H_5$ ; $R^4 = H$,
i. $R^1 = H$ ; n = m = 1 ; $R^2 = R^3 = CH_3$ ; $R^4 = H$.

and wherein one of the dotted lines indicates a carbon-carbon single bond and the other one a double bond, or each of them indicates a single bond.

2. (-)-(E)-3,3-Dimethyl-5-(2',2',3'-trimethyl-3'-cyclopenten-1'-yl)-4-penten-2-ol.
3. (-)-(1'S,E)-3-Ethyl-3-methyl-5-(2',2',3'-trimethyl-3'-cyclopenten-1'-yl)-4-penten-2-ol.
4. (+)-(1'S)-2-Methyl-4-(2',2',3'-trimethyl-3'-cyclopenten-1'-yl)-1-pentanol.
5. (-)-(1'R)-2-Methyl-4-(2',2',3'-trimethyl-3'-cyclopenten-1'-yl)-4-hexen-1-ol.
6. (-)-(1'S)-2-Methyl-4-(2',2',3'-trimethyl-3'-cyclopenten-1'-yl)-4-penten-1-ol.
7. (-)-(1'S,E)-2-Ethyl-2-methyl-4-(2',2',3'-trimethyl-3'-cyclopenten-1'-yl)-3-buten-1-ol.
8. (-)-(1'S,E)-2,2-Dimethyl-4-(2',2',3'-trimethyl-3'-cyclopenten-1'-yl)-3-buten-1-ol.
9. (-)-(1',S)-2-Ethyl-2-methyl-4-(2',2',3'-trimethyl-3'-cyclopenten-1'-yl)-1-butanol.
10. (+)-2,2-Dimethyl-4-(2',2',3'-trimethyl-3'-cyclopenten-1'-yl)-1-butanol.
11. Utilization of one of the compounds according to claim 1 as perfuming ingredient for the preparation of perfumes and perfumed products.
12. Perfuming composition resulting from the utilization according to claim 11.

**Patentansprüche**

1. Verbindungen der Formel

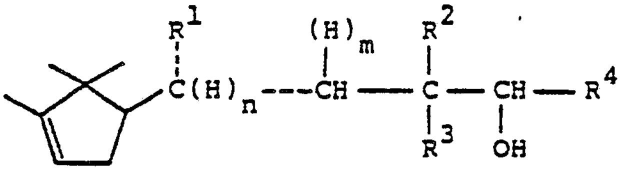

(I)

in welcher :

a. $R^1 = CH_3$ ; n = m = 1 ; $R^2 = CH_3$ ; $R^3 = R^4 = H$,
b. $R^1 = CH_2$ ; n = 0 ; m = 1 ; $R^2 = CH_3$ ; $R^3 = R^4 = H$,
c. $R^1 = CH\text{-}CH_3$ ; n = 0 ; m = 1 ; $R^2 = CH_3$ ; $R^3 = R^4 = H$,
d. $R^1 = H$ ; n = m = 0 ; $R^2 = R^4 = CH_3$ ; $R^3 = C_2H_5$,
e. $R^1 = H$ ; n = m = 0 ; $R^2 = R^3 = R^4 = CH_3$,
f. $R^1 = H$ ; n = m = 0 ; $R^2 = CH_3$ ; $R^3 = C_2H_5$ ; $R^4 = H$,
g. $R^1 = H$ ; n = m = 0 ; $R^2 = R^3 = CH_3$ ; $R^4 = H$,
h. $R^1 = H$ ; n = m = 1 ; $R^2 = CH_3$ ; $R^3 = C_2H_5$ ; $R^4 = H$,
i. $R^1 = H$ ; n = m = 1 ; $R^2 = R^3 = CH_3$ ; $R^4 = H$.

und in welcher eine der gestrichelten Linien eine Kohlenstoff-Kohlenstoff Einfachbindung und die andere eine Doppelbindung, oder beide eine Einfachbindung darstellen.

2. (-)-(E)-3,3-Dimethyl-5-(2',2',3'-trimethyl-3'-cyclopenten-1'-yl)-4-penten-2-ol.
3. (-)-(1'S,E)-3-Ethyl-3-methyl-5-(2',2',3'-trimethyl-3'-cyclopenten-1'-yl)-4-penten-2-ol.
4. (+)-(1'S)-2-Methyl-4-(2',2',3'-trimethyl-3'-cyclopenten-1'-yl)-1-pentanol.
5. (-)-(1'R)-2-Methyl-4-(2',2',3'-trimethyl-3'-cyclopenten-1'-yl)-4-hexen-1-ol.
6. (-)-(1'S)-2-Methyl-4-(2',2',3'-trimethyl-3'-cyclopenten-1'-yl)-4-penten-1-ol.
7. (-)-(1'S,E)-2-Ethyl-2-methyl-4-(2',2',3'-trimethyl-3'-cyclopenten-1'-yl)-3-buten-1-ol.
8. (-)-(1'S,E)-2,2-Dimethyl-4-(2',2',3'-trimethyl-3'-cyclopenten-1'-yl)-3-buten-1-ol.
9. (-)-(1',S)-2-Ethyl-2-methyl-4-(2',2',3'-trimethyl-3'-cyclopenten-1'-yl)-1-butanol.
10. (+)-2,2-Dimethyl-4-(2',2',3'-trimethyl-3'-cyclopenten-1'-yl)-1-butanol.
11. Verwendung einer der Verbindungen gemäss Anspruch 1 als Riechstoff zur Herstellung von Parfümen und parfümierten Produkten.
12. Riechstoff Komposition die aus der Verwendung gemäss Anspruch 11 resultiert.